# EUROPEAN PATENT APPLICATION

(11) **EP 1 375 513 A1**
(43) Date of publication of application: **02.01.2004**
(21) Application number: 02702879.4
(22) Date of filing: 11.03.2002
(51) Int. Cl.: C07K 14/47, A61K 38/17, A61P 37/04, C07K 5/08, C07K 7/06, C07K 7/08, A23J 3/34, A23L 1/305

(54) **IMMUNOPOTENTIATORS**

(30) Priority: 09.03.2001 JP 2001067600
(71) Applicant: Meiji Seika Kaisha, Ltd., Tokyo 104-8002 (JP)
(72) Inventor: OTANI, Hajime Faculty of Agriculture, Shinshu Univ, Kamiina-gun, Nagano 399-4598 (JP); TASHIRO, Y., New Materials Meiji Seika Kaisha Ltd, Sawai-ku Kawasaki (JP); SUGIHARA, M., New Materials Meiji Seika Kaisha Ltd, Saiwai-ku Kawasa (JP)
(74) Representative: Gillard, Richard Edward
(86) International application number: PCT/JP2002/002258
(87) International publication number: WO 2002/072629

(57) **Abstract**

An object of the present invention is to provide a peptide with superior immuno-enhancing activities, which comprises particular amino acid sequence, and an immuno-enhancing agent comprising such a peptide. The present invention relates to the immuno-enhancing agent comprising a peptide consisting of the following amino acid sequence (I): Q1-SerP-X-SerP-Q2 (I); wherein, SerP represents the phosphoserine residue, X represents one to three of any amino acid residues, and either of Q1 or Q2 dose not exist or neither of them exists, or Q1 and Q2 are independently at least one of any amino acid residue.

SEQUENCE LISTING

<110> MEIJI SEIKA KAISHA, LTD.

<120> Immuno potentiator

<130> 13012401

<140>

<141>

<160> 13

<170> PatentIn Ver. 2.1

<210> 1

<211> 8

<212> PRT

<213> Artificial Sequence

<220>

<223> Description of Artificial Sequence: synthetic
peptide; In the sequence, "Ser" is modified by
phosphoric acid, and "Xaa" is represented 1 to 3
any amino acids.

<400> 1

Glu Ser Xaa Ser Ser Ser Glu Glu
1               5

<210> 2

<211> 5

<212> PRT

<213> Artificial Sequence

<220>

<223> Description of Artificial Sequence: synthetic
      peptide; In the sequence, "Ser" is modified by
      phosphoric acid, and "Xaa" is represented 1 to 3
      any amino acids.
<400> 2
Glu Ser Xaa Ser Ser
 1               5

<210> 3
<211> 5
<212> PRT
<213> Artificial Sequence
<220>
<223> Description of Artificial Sequence: synthetic
      peptide; In the sequence, "Ser" is modified by
      phosphoric acid, and "Xaa" is represented 1 to 3
      any amino acids.
<400> 3
Ser Xaa Ser Glu Glu
 1               5

<210> 4
<211> 5
<212> PRT
<213> Artificial Sequence
<220>
<223> Description of Artificial Sequence: synthetic

peptide; In the sequence, "Ser" is modified by
phosphoric acid, and "Xaa" is represented 1 to 3
any amino acids.
<400> 4
Glu Ser Xaa Ser Glu
1               5

<210> 5
<211> 8
<212> PRT
<213> Artificial Sequence
<220>
<223> Description of Artificial Sequence: synthetic
peptide; In the sequence, "Ser" is modified by
phosphoric acid.
<400> 5
Glu Ser Leu Ser Ser Ser Glu Glu
1               5

<210> 6
<211> 5
<212> PRT
<213> Artificial Sequence
<220>
<223> Description of Artificial Sequence: synthetic
peptide; In the sequence, "Ser" is modified by
phosphoric acid.

<400> 6
Glu Ser Leu Ser Ser
1               5

<210> 7
<211> 5
<212> PRT
<213> Artificial Sequence
<220>
<223> Description of Artificial Sequence: synthetic
      peptide; In the sequence, "Ser" is modified by
      phosphoric acid.
<400> 7
Ser Ser Ser Glu Glu
1               5

<210> 8
<211> 5
<212> PRT
<213> Artificial Sequence
<220>
<223> Description of Artificial Sequence: synthetic
      peptide; In the sequence, "Ser" is modified by
      phosphoric acid.
<400> 8
Glu Ser Leu Ser Glu
1               5

<210> 9
<211> 25
<212> PRT
<213> Artificial Sequence
<220>
<223> Description of Artificial Sequence: synthetic
peptide; In the sequence, "Ser" is modified by
phosphoric acid, and "Xaa" is represented 1 to 3
any amino acids.
<400> 9
Arg Glu Leu Glu Glu Leu Asn Val Pro Gly Glu Ile Val Glu Ser Xaa
1               5                   10                  15

Ser Ser Ser Glu Glu Ser Ile Thr Arg
            20                  25

<210> 10
<211> 25
<212> PRT
<213> Bos sp.
<400> 10
Arg Glu Leu Glu Glu Leu Asn Val Pro Gly Glu Ile Val Glu Ser Leu
1               5                   10                  15

Ser Ser Ser Glu Glu Ser Ile Thr Arg
            20                  25

<210> 11
<211> 28
<212> PRT
<213> Artificial Sequence
<220>
<223> Description of Artificial Sequence: synthetic
peptide; In the sequence, "Ser" is modified by
phosphoric acid, and "Xaa" is represented 1 to 3
any amino acids.
<400> 11
Arg Glu Leu Glu Glu Leu Asn Val Pro Gly Glu Ile Val Glu Ser Leu
1               5                   10                  15

Ser Ser Ser Glu Glu Ser Ile Thr Xaa Ile Asn Lys
            20                  25

<210> 12
<211> 28
<212> PRT
<213> Bos sp.
<400> 12
Arg Glu Leu Glu Glu Leu Asn Val Pro Gly Glu Ile Val Glu Ser Leu
1               5                   10                  15

Ser Ser Ser Glu Glu Ser Ile Thr Arg Ile Asn Lys
            20                  25

<210> 13
<211> 8
<212> PRT
<213> Artificial Sequence
<220>
<223> Description of Artificial Sequence:  synthetic
      peptide
<400> 13
Glu Ser Leu Ser Ser Ser Glu Glu
  1               5

## Description

### BACKGROUND OF THE INVENTION

### Field of the Invention

The present invention relates to an immuno-enhancing agent that comprises particular peptides.

### Background of the Art

Milk is nutrition source by itself, and popularly taken. Casein, which is protein included in milk, is also traditionally utilized as a protein source for the modified milk for infants or specific modified milk, because casein is easily digested and the essential amino acids taken from it is well balanced.

In general, caseins contain the amino acid residue, phosphoserine, which has phosphoric acid group and is less included in usual protein. From the enzyme-digested casein fractions, casein phosphopeptide (referred to as "CPP" hereinafter) is obtained by isolating and purifying the phosphoserine residue-rich fractions.

It is known that CPP has the role to keep calcium in soluble form, in which it turns insoluble form in the small intestine, and promotes calcium absorption. Therefore, CPP is utilized in many kinds of foods and raw material for feeds. It is also known that CPP has the activity to enhance immunoglobulin A production in cell cultures or in digestive tracts of animals (for example, Milk Science vol. 49(2000), pp73-79; and Food and Agricultural Immunology, 12(2000), pp 165-173).

Since CPP is obtained by enzymatically digesting casein, its antigenicity is lower than casein itself, but the antigenicity is not completely loosen. Therefore, when a person who has an allergy to milk casein takes CPP with expectation of enhancing his/her immunity, there is an anxiety that he/she has the allergic reaction caused by CPP.

Alternatively, CPP is described, for example, in WO 96/29340, which discloses that CPP promotes to absorb minerals in the small intestine. However, there is no description for the immuno-enhancing activity from CPP.

### SUMMARY OF THE INVENTION

The present inventors have now found that, among the peptides derived from casein, peptides having the specific amino acid sequences in which plural phosphoserine residues are included show the excellent immuno-enhancing activity. These peptides show strong immuno-enhancing activity even though their molecular weights are relatively low. The present invention has been made based on such finding.

Accordingly, it is an object of the present invention to provide the peptides comprising the specific amino acid sequences, which have excellent immuno-enhancing activities, and the immuno-enhancing agent comprising such peptides.

According to one embodiment of the present invention, there is provided an immuno-enhancing agent comprising the peptides consisting of the following amino acid sequence (I): Q1-SerP-X-SerP-Q2 (I) wherein,
SerP represents a phosphoserine residue,
X represents 1 to 3 of any amino acid residues, and
one of Q1 and Q2 dose not exist or neither of them are exists, or Q1 and Q2 are independently at least one of any amino acid residue.

A peptide according to the present invention comprises the following amino acid sequence (Ia): Q1-SerP-X-SerP-Q2     (Ia) wherein,
SerP represents a phosphoserine residue,
X represents 1 to 3 of any amino acid residues,
Q1 represents 0(zero) to 14 of any amino acid residues, and
Q2 represents 0(zero) to 8 of any amino acid residues, excluding the case where the number of amino acid in Q2 is 8 and the number of amino acid in Q1 is 14.

Preferably, the amino acid sequence (I) according to the present invention has an immuno-enhancing activity.

The immuno-enhancing agent of the present invention is excellent in the immuno-enhancing activities such as the mitogenicity of spleen cells and the enhancement activity of the immunoglobulin production. The peptide consisting of the amino acid sequence (I) contained in the present immuno-enhancing agent has the low molecular weight peptide including a plurality of phosphoserine molecules and has the immuno-enhancing activity. The peptide is apparently different substance from CPP, which is conventionally known to have the immuno-enhancing activity and its molecular weight is about 3,000 to 5,000. Therefore, the peptide consisting of the amino acid sequence (I) of the present invention has the excellent immuno-enhancing activity, as well as the milk allergy reaction caused by the peptide is drastically reduced compared with those caused by using the casein or CPP, or such an allergy reaction is not caused by the peptide of the present invention. Therefore, the immuno-enhancing agent of the present invention is superior in safety.

The immuno-enhancing agent according to the present invention is useful as the raw material for foods or medicament to bring the immuno-enhancing action on a person whose immunity is weak. For example, when the immuno-enhancing agent of the present invention is given to an infant, it is expected that the immunity of the infant would be enhanced. The transferring antibody from mother's milk prevents the infant immediately after its birth from various infections, and the transferring antibody in the mother's milk is mainly composed of immunoglobulin A. Therefore, when a mother who cannot give her milk to her baby feeds the immuno-enhancing agent to her baby, it is expected that the similar effect to that derived from being given the mother's milk to the baby would be brought. Since the immuno-enhancing agent of the present invention can be composed of the low molecular weight peptides lacking for antigenicity, the mother can give it to her baby or infant having the milk allergy without any anxiety. Furthermore, even in an adult with decreased immunity caused by aging, several diseases, fatigue and so forth, they can promote their health daily in safe if they use the immuno-enhancing agent of the present invention.

### BRIEF DESCRIPTION OF THE DRAWINGS

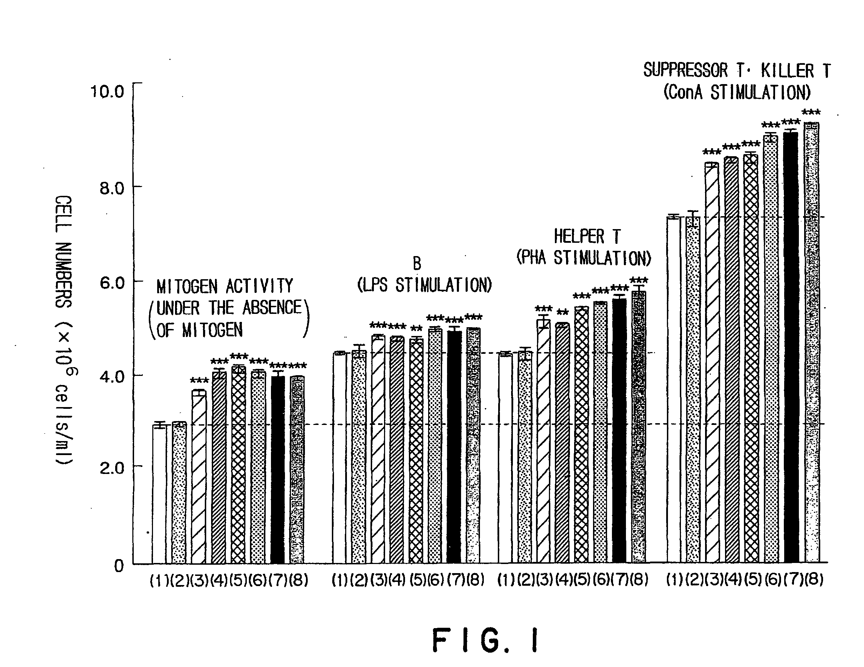

Fig. 1 shows the result of the effect to the proliferation activities of murine spleen cells when the control sample and Peptides ((1) to (8)) prepared in Example 1 are added respectively. In this figure, the number of (1) to (8) respectively correspond to Peptides (1) to (8) prepared in Example 1. In the figure, the asterisk super scribed (*) shows that the sample has statistically significant differences to control sample, and the double asterisks super scribed (**) means P < 0.01 and the triple asterisks super scribed (***) means P < 0.001.

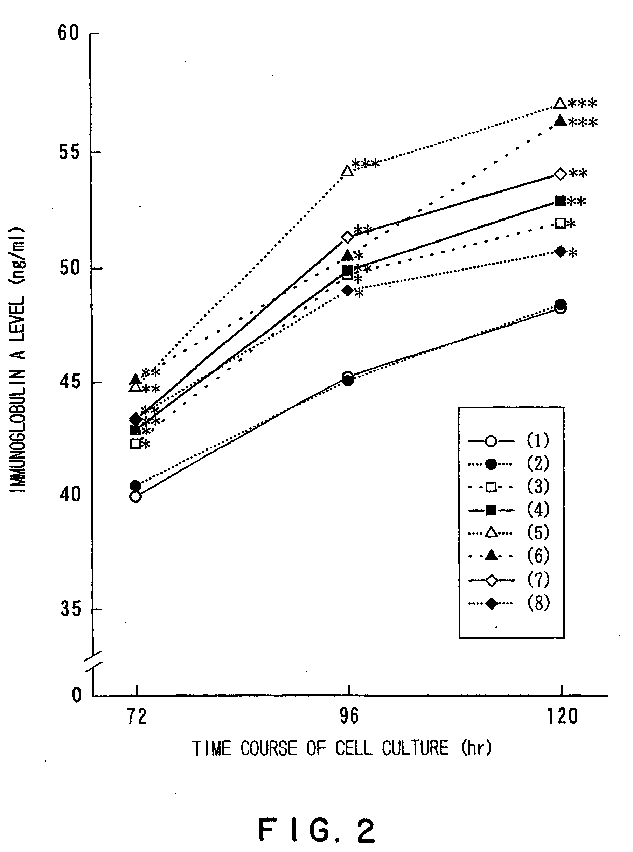

Fig. 2 shows the effect to the IgA production in the murine spleen cell culture system when the control sample and Peptides ((1) to (8)) prepared in Example 1 are added respectively. In this figure, the number of (1) to (8) respectively correspond to Peptides (1) to (8) prepared in Example 1. In the figure, the asterisk super scribed (*) shows that the sample have statistically significant differences to the control sample; the single asterisk super scribed (*) means P < 0.05, the double asterisks super scribed (**) means P < 0.01 and the triple asterisks super scribed (***) means P < 0.001.

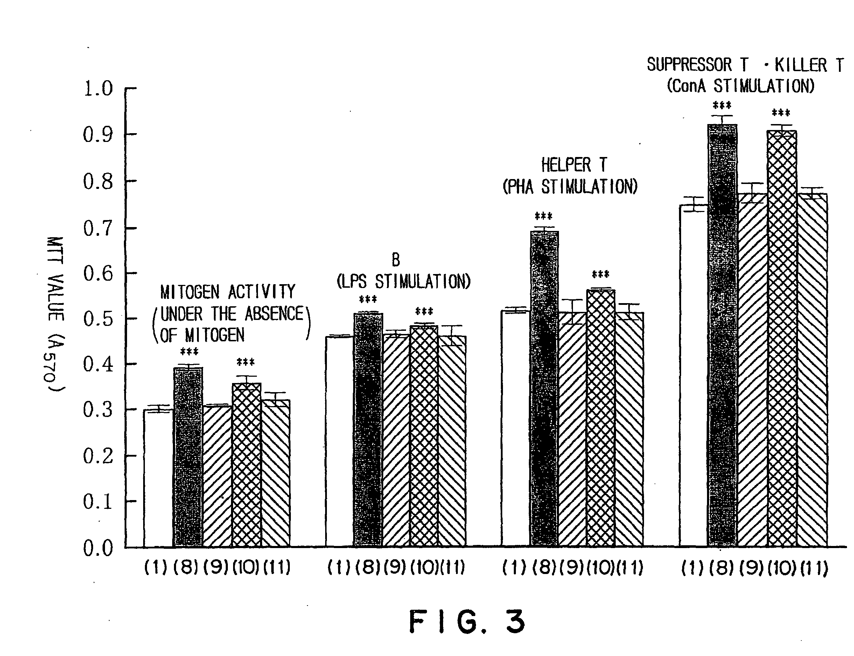

Fig. 3 shows the effect to the proliferation activity of the murine spleen cells when Peptides (8) to (11) and the control sample (1) prepared in Example 1 are respectively added. In this figure, both the numbers (1) and (8) respectively correspond to Peptides (1) and (8) prepared in Example 1, and the numbers (9) to (11) respectively correspond to those (9) to (11) prepared in Example 2. In the figure, the asterisk super scribed (*) shows that the sample has statistically significant differences to control sample, and the triple asterisks super scribed (***) means P < 0.001.

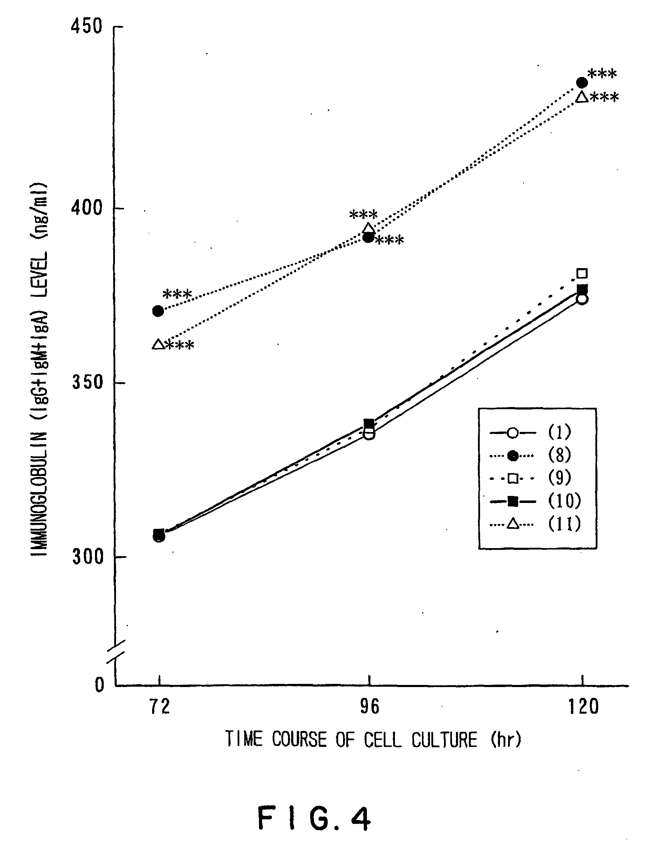

Fig. 4 shows the effect to the immunoglobulin (IgG + IgM + IgA) production in the murine spleen cell culture system when Peptides (8) to (11) and the control sample (1) prepared in Example 2 are added respectively. In this figure, the both numbers (1) and (8) respectively correspond to Peptides (1) and (8) prepared in Example 1, and the numbers (9) to (11) respectively correspond to those (9) to (11) prepared in Example 2. In the figure, the asterisks super scribed (*), shows that the sample has statistically significant differences to the control sample, and the single asterisk (*) means P < 0.05, the double asterisks super scribed (**) means P < 0.01 and the triple asterisks super scribed (***) means P < 0.001.

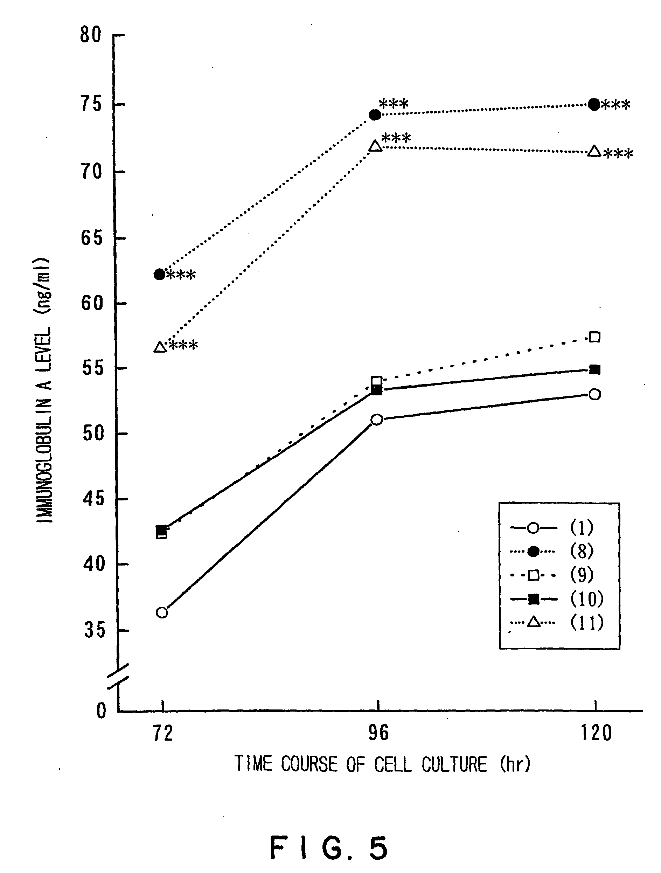

Fig. 5 shows the effect to the IgA production in the murine spleen cell culture system when Peptides (8) to (11) and the control sample (1) prepared in Example 2 are added respectively. In the figure, the both numbers (1) and (8) respectively correspond to Peptides (1) and (8) prepared in Example 1, and the numbers (9) to (11) respectively correspond to those (9) to (11) prepared in Example 2. Furthermore, the asterisk super scribed (*) shows that the sample has statistically significant differences to the control sample, and the single asterisk (*) means P < 0.05, the double asterisks super scribed (**) means P < 0.01 and the triple asterisks super scribed (***) means P < 0.001.

### DETAILED EXPLANATION OF THE INVENTION

### Peptides

An immuno-enhancing agent according to the present invention comprises a peptide consisting of at least the amino acid sequence "SerP-X-SerP" as a basic structure. Therefore, the immuno-enhancing agent of the present invention comprises a peptide consisting of the following amino acid sequence (I): Q1-SerP-X-SerP-Q2 (I) wherein,
SerP represents a phosphoserine residue,
X represents 1 to 3 of any amino acid residue, and
either of Q1 and Q2 dose not exist or neither of them exists, or Q1 and Q2 are independently at least one of any amino acid residue.

The term "immuno-enhancing agent" herein means an agent having the immuno-enhancing activity, and its application is not limited to pharmaceuticals, but it may be used for foods or feeds and so forth.

The term "to have the immuno-enhancing activity" herein means that it enables to enhance the immuno-enhancing activities such as the mitogen activity or the promotion of the immunoglobulin production by the spleen cells from mammals, especially human. For example, when the activity is determined under the same condition as that described in Example 1 of the present specification, it means that the evaluation shows that the immuno-enhancing activity is significantly recognized by using the enhancement of the spleen cell proliferation and the increase of the immunoglobulin content as indexes.

The "amino acids" herein includes the isomers of them, that is, both the D-form and L-form of them. The amino acid in the specification may includes not only 20 of the amino acids which compose of natural proteins, but also other amino acids such as α-amino acid, β-amino acid, γ-amino acid, δ-amino acid, and non-natural amino acids.

According to a preferred embodiment of the present invention, in the amino acid sequence (I), X consists of one of any amino acids.

When X in the amino acid sequence (I) represents any one amino acid, X is preferably selected form the group consisting of Leu, Glu, Ile, Thr, Ala, Arg, Lys, Asn, His, Val, and SerP. More preferably, X is SerP or Leu, and most preferably, X is SerP.

In the amino acid sequence (I), when Q1 exists, Q1 represents at least one of any amino acids. Preferably, the number of the amino acid residues represented by Q1 is 1 to 201, more preferably 1 to 128, further preferably 1 to 45, furthermore preferably 1 to 23, and specifically preferably 1 to 14.

When Q2 exists in the sequence (I), Q2 represents at least one of any amino acids. Preferably, the number of the amino acid residue represented by Q2 is 1 to 203, more preferably 1 to 192, further preferably 1 to 151, furthermore preferably 1 to 76, specifically preferably 1 to 31, and the most preferably 1 to 11.

According to one preferred embodiment of the present invention, in the amino acid sequence (I), the number of the amino acid residue represented by Q1 is 0 to 14, and that represented by Q2 is 0 to 8. More preferably, the number of the amino acid residue represented by Q1 is 0 to 8, and that represented by Q2 is 0 to 5. Furthermore preferably, the number of the amino acid residue represented by Q1 is 0 to 1, and that represented by Q2 is 0 to 2.

Further, in the peptides used for the immuno-enhancing agent according to the present invention, preferably, the number of the amino acid residue represented by Q1 is 14 and that represented by Q2 is 8 is excepted.

According to other embodiment of the present invention, there is provided a new peptide represented by the following sequence (Ia): Q1-SerP-X-SerP-Q2 (Ia) wherein,
SerP represents a phosphoserine residue,
X represents 1 to 3 of any amino acid residues,
Q1 represents 0 to 14 of any amino acid residues, and
Q2 represents 0 to 8 of any amino acid residues, excluding the case where the number of amino acid in Q2 is 8 and the number of amino acid in Q1 is 14.

The new peptide may be identical to the peptides consisting of the amino acid sequence (I) as long as it is included in said sequence (Ia).

According to another preferred embodiment of the present invention, neither the amino acid sequence represented by Q1 nor Q2 exists in said amino acid sequence (I). That is, said amino acid sequence (I) is preferably the oligopeptide being composed of "SerP-X-SerP". When the number of the amino acid in the amino acid sequence (I) is such small number, the peptide does not show the antigenicity. Therefore, the immuno-enhancing agent of the present invention does not cause any allergic reaction, when it is used. Accordingly, the highly safe immuno-enhancing agent can be obtained.

According to more preferred embodiment of the present invention, said amino acid sequence (I) is selected from the group consisting of: Glu-SerP-X-SerP-SerP-SerP-Glu-Glu  (SEQ. ID No: 1); Glu-SerP-X-SerP-SerP (SEQ. ID No: 2); 
SerP-X-SerP-Glu-Glu (SEQ. ID No: 3);
SerP-X-SerP; and
 Glu-SerP-X-SerP-Glu (SEQ. ID No: 4). More preferably, it is SerP-X-SerP.

According to further preferred embodiment of the present invention, said amino acid sequence (I) is selected from the group consisting of: Glu-SerP-Leu-SerP-SerP-SerP-Glu-Glu   (SEQ. ID No: 5); Glu-SerP-Leu-SerP-SerP (SEQ. ID No: 6); 
SerP-SerP-SerP-Glu-Glu (SEQ. ID No: 7);
SerP-Leu-SerP;
SerP-SerP-SerP; and
 Glu-SerP-Leu-SerP-Glu (SEQ. ID No: 8). More preferably, it is SerP-Leu-SerP or SerP-SerP-SerP.

According to another preferred embodiment of the present invention, said amino acid sequence (I) is 
Arg-Glu-Leu-Glu-Glu-Leu-Asn-Val-Pro-Gly-Glu-Ile-Val-Glu-
SerP-X-SerP-SerP-SerP-Glu-Glu-Ser-Ile-Thr-Arg (SEQ. ID
No: 9),
 and
more preferably it is

Arg-Glu-Leu-Glu-Glu-Leu-Asn-Val-Pro-Gly-Glu-Ile-Val-Glu-
SerP-Leu-SerP-SerP-SerP-Glu-Glu-Ser-Ile-Thr-Arg (SEQ. ID
No: 10).

According to more another preferred embodiment of the present invention, said amino acid sequence (I) is 
Arg-Glu-Leu-Glu-Glu-Leu-Asn-Val-Pro-Gly-Glu-Ile-Val-Glu-
SerP-Leu-SerP-SerP-SerP-Glu-Glu-Ser-Ile-Thr-Y-Ile-Asn-
Lys          (SEQ. ID No: 11),
 wherein, Y represents any one amino acid residue, and it is preferably Arg or Cys, and
further preferably, it is

Arg-Glu-Leu-Glu-Glu-Leu-Asn-Val-Pro-Gly-Glu-Ile-Val-Glu-
SerP-Leu-SerP-SerP-SerP-Glu-Glu-Ser-Ile-Thr-Arg-Ile-Asn-
Lys          (SEQ. ID No: 12).

In the present invention, the term "peptide" includes its derivatives. The term of the "derivatives of the peptide" herein means those having said immuno-enhancing activity, and means that in the parts in said peptides other than "SerP-X-SerP" which is sequence of a basic structure, a part of or all of amino groups of either the amino acids at their amino terminal (N-terminal) or the side chain of the amino acids; and/or a part of or all of carboxyl groups of either the amino acids at carboxyl terminal (C-terminal) or the side chain of the amino acids; and/or a part of or all of functional groups except an amino group and a carboxyl group of the side chain of the amino acids of the peptide, for example, a hydrogen, a thiol group, an amino group and the like are modified by other suitable subustituents. Such modifications by other substitutes may be performed in order to protect the functional groups which are present in the peptides, to improve the safety and transition into the tissues of the peptide, or to enhance their activities or the like. Accordingly, the present invention includes the derivatives of the peptides represented by the above-mentioned sequence ID Nos. 1 to 12.

According to a preferred embodiment of the present invention, the peptide of the present invention comprises a repetitive sequence in which the above-mentioned amino acid sequence (I) is repeated at least twice.

The number of repeat of the amino acid sequence (I) in the peptide of the present invention is at least twice in this case, preferably 2 to 70, more preferably 2 to 30, further preferably 2 to 10, furthermore preferably 2 to 4. The greater number of repeat is preferable to attain the more remarkable above-mentioned effectiveness. However, from the viewpoint of the increase of the costs caused by the complicated repeating process to produce the peptide having repeat sequence, the safety or convenience when the peptide is used as the immuno-enhancing agent, said number of the repeat is preferably 2 to 10, more preferably 2 to 4.

According to one preferred embodiment of the present invention, when the peptide of the present invention comprises a repetitive sequence in which the above-mentioned amino acid sequence is contained at least twice, the number of the amino acid residue composed of the peptide is preferably equal to or less than 224, more preferably equal to or less than 100, further preferably equal to or less than 30.

### A method for producing the peptide

The peptide according to the present invention may be produced by using the various synthetic method conventionally used or obtained from the natural sources. Since the amino acid sequence of the peptide of the present invention is sequenced, the whole peptide may be synthesized, or a partial peptide of that is obtained from the natural sources to synthesize the whole peptide.

When the peptide of the present invention is obtained from the natural source, it may be obtained according to the following procedure.

At first, milk casein as casein or yolk protein is provided, wherein the casein or protein includes the protein comprising some phosphoserines such as αs1-casein, αs2-casein, β-casein, and phosvitin.

Then, casein or yolk protein is hydrolyzed by the so-called trypsin treatment under the conventional condition, and the supernatant obtained is collected to obtain casein phosphopeptide (CPP). The specific example of such a CPP preparation includes the following methods (1) to (3):
(1) a method comprising the steps of hydrolyzing β-casein by using crystallized trypsin to generate CPP, precipitating the unreacted casein and the partial impurities at pH 4.7 to remove, and adding barium chloride and ethanol to the supernatant so as to become their final concentration 50 % (v/v) to collect CPP as the precipitate (for example, R. F. Peterson, L. W. Norman and T. L. McMeekin, Journal of the American Chemical Society, 80, pp95-99 (1958));
(2) a method comprising the steps of hydrolyzing (-casein by using crystallized trypsin to generate CPP, treating it by using trichloroacetic acid to remove insoluble substances, neutralizing the solution by using sodium hydroxide to adjust pH 5.0, partially fractionating by using a gel filtration column, Sephadex G-25R (manufactured by Pharmacia Co.), adsorbing on an ion-exchange column, Dowex 50x2R (manufactured by Dow Chemical Co.), eluting by using pyridine-acetic acid buffer (0.2 N, pH 2.5) to purify CPP (H. Naito and H. Suzuki, Agricultural Biological Chemistry, 38, pp1543-1545 (1974)); and
(3) a method comprising the steps of hydrolyzing casein by using trypsin under the condition of such a desired temperature and pH, for example, pH 7.0 to 8.5 and at 50 °C, to generate CPP, precipitating the unreacted casein and the partial impurities around pH 5 to remove them, adding calcium salt or calcium acetate with an organic solvent such as ethanol to the supernatant to collect CPP as the precipitate (Japanese patent examined publication H02-7616).

The peptide of the present invention having the desired length for the application may be obtained by treating CPP obtained as mentioned above with the desired combination of endoprotease such as trypsin and chymotrypsin and the various peptidase such as aminopeptidase and carboxypeptidase.

Therefore, according to another embodiment of the present invention, there is provided a method for producing said peptide, wherein the method comprises the steps of:
providing casein,
treating the casein with trypsin, and precipitating by adding calcium salt and ethanol to obtain phosphopeptide, and then
treating the phosphopeptide with an enzyme selected from the group consisting of aminopeptidase, carboxypeptidase and the combination thereof to obtain the peptide.

Alternatively, the peptide according to the present invention may be obtained by using the following procedures when it is obtained by utilizing various kinds of synthetic method commonly used.

Briefly, the peptide of the present invention may be prepared in accordance with the conventional method, for example, the method of Paquet et al. (A. Paquet and M. Johns, Int. J. Pept. Protein Res., 36(2), pp97-103, (1990)), applying the general method for synthesizing peptides. Showing the example, firstly, phosphoserine protected the parts of an amino acid by t-butoxy carbonyl (Boc) group or the like, and any amino acids are combined by using conventional methods for synthesizing the peptide such as the activation ester method and condensation method to elongate the length of the amino acid in stepwise, thereby the phosphopeptide including phosphoserine residue at desired positions can be prepared. This method can be carried out in both the liquid phase and the solid phase.

### Immuno-enhancing agent

The immuno-enhancing agent according to the present invention can be used in the use as pharmaceuticals, foods or feeds.

Therefore, according to one preferred embodiment of the present invention, there is provided the immuno-enhancing agent used as a pharmaceutical. More preferably, the immuno-enhancing agent is used as the pharmaceutical for enhancing immunoglobulin production.

When the immuno-enhancing agent of the present invention is used as the pharmaceutical, it can be formed to dosage forms suitable to administration routes. Specifically, it can be mainly prepared to any one of the dosage forms as follows: injectable dosage forms such as intravenous injection or intra muscle injection, agents for oral administration such as capsules, coating tablets, drops, tablets, granules, powder, pills, fine granules, sugar-coated tablets, and troches, agents for rectal administrations, and oleaginous suppository. If necessary, the immune-enhancing agent can be the form of liquids or capsules in which liquid preparation is included.

These pharmaceuticals can be produced in accordance with the conventional method by using the pharmaceutically acceptable additives such as vehicles, filling agents, binders, moistening agents, disintegrators, surface active agents, lubricants, emulsifiers, buffers, preservatives, solubilizing agents, antiseptis, corrigents, soothing agents, and stabilizers. That is, the immuno-enhancing agent of the present invention can further comprise the pharmaceutically acceptable additives for pharmaceuticals.

The additives for the pharmaceuticals which can be used in the present invention includes lactose, fructose, glucose, starch, gelatin, magnesium carbonate, synthetic magnesium silicate, talc, magnesium stearate, methylcellulose, or salts thereof, acacia gum, polyethyleneglycol, syrup, petrolatum, glycerin, ethanol, propylene glycol, citric acid, sodium chloride, sodium sulfite, and sodium phosphate.

When the immuno-enhancing agent of the present invention is used as the pharmaceutical, various types of route are taken as its administration route, and such routes include the oral administration, the parenteral administration, inhalation, rectal administration, and local administration. Among them, parenteral administration includes subcutaneous injection, intravenous injection, intramuscular injection, intranasal administration or intranasal injection. For example, the administration can be carried out via mucosa such as tunica mucosa nasi, tunica mucosa oris, baccal mucosa, and rectal mucosa or percutaneous, or by embedding pharmaceuticals. Accordingly, the immuno-enhancing agent of the present invention can be administrated via any of the route as mentioned above to the patients selected from the group consisting of humans and non-human animals.

Suitable administration dosage is determined by considering the direction for use, the age and the sexuality of the patient, the grave of the symptoms appeared. The dosage is preferable about 10 to 1,000 mg per a day per adult in general, more preferably 50 to 500 mg. The administration is carried out at single dose or multiple doses per a day.

Thus, the immuno-enhancing agent of the present invention which is the pharmaceutical form can be advantageously used in the treatment or prophylaxis of the disease such as various infectious diseases, or the treatment of allergies or autoimmune disease.

Namely, it is known that the secretory antibody, IgA, which is immunoglobulin secreted in milk, inhibits that the microorganisms having strong pathogenicity bind to the mucosa of the intestinal tract, and inactivates the bacterial toxins by specifically binding to the toxins. It is also known that IgA suppresses the allergic reaction by binding to dietary antigens that act as allergens thereby preventing them to pass through paries of the digestive tracts. On the other hand, it is known that in the infant grown with the artificial feeding, i.e., the powdered milk for child-rearing which has no secretory IgA, and the patient with IgA deficiency disease, IgG antibody against the dietary antigen appears more frequently, and the allergic disease or the autoimmune disease is shown more frequently. The immuno-enhancing agent of the present invention can promote the induction of IgA having effects to prevent the infection or to suppress the allergic reaction in the body so that the immunity of the body can be enhanced.

When the immuno-enhancing agent of the present invention is used as foods, it can be provided as the form of gums, biscuits, chocolates, candies, jellies, tablet type of the confectioneries, powdered milk, beverages and the like.

That the immuno-enhancing agent of the present invention is provided in the form of the food is advantageous for supplying the nutrients to the infant, old person, or the convalescent who are inferior to the healthy adult in physical strength, and for improving the health of them. Furthermore, it is also advantageous to improve the health of the patient with the disease such as allergic disease.

When the immuno-enhancing agent of the present invention is used as feeds, it is provided in the form of feeds for domestic animals such as the cow, the swine, the poultry and so forth, the pet feed, various types of combined feeds and the like.

Suitable supplying dosage is determined by considering the direction for the age, the weight, and the sexuality of the animal, as well as the circumstances for feeding. The content of immuno-enhancing agent in the feed is preferable about 0.01 to 1 % of the immuno-enhancing agent by weight based on entire weight of the feed to be supplied, more preferable about 0.05 to 0.5 % by weight. Such feeds are continuously fed to the animal such as a young pig for a certain term, preferably more than 3 weeks, more preferably around 5 weeks.

The immuno-enhancing agent of the present invention provided in the form of the feed can conveniently enhance the immunity of the domestic animals. Therefore, it can intend to treat or prevent the infectious disease of the domestic animal, for example, such as the cow in a meadow or the allergic disease.

Alternatively, by utilizing the immuno-enhancing agent of the present invention as the feed, the amount of the immuno-enhancing agent used in the feed is less than that of CPP thereby the ratio of other components in the feed can be increased.

According to another embodiment of the present invention, there is provided the method for enhabcing the immunity of a patient, wherein the method comprises administrating the peptide of the present invention to the patient with the decreased immunity or the weak immunity.

According to the other embodiment of the present invention, use of the peptide according to the present invention for producing the immuno-enhancing agent is provided.

### EXAMPLES

The following examples further illustrate the present invention, but should not be construed as limiting the scope of the present invention.

### Example 1

### Preparation of the peptide:

In order to identify an active center of the position from 1 to 28 of β-casein, the following experiment was performed.

β-casein of which the purity was higher than 90 % (available from Sigma Chemical Co.) was dissolved in water to prepare a 15 % solution (w/w), and trypsin (manufactured by Novo Inc.) was added into the solution. This solution was reacted for 2 hours under the condition of pH 7.0 at 60 °C. Then, the impurities were precipitated from the solution by charging the pH value to 4.5 to be removed. Calcium was added to the obtained supernatant so as to be 1.1 % volume of the supernatant, and ethanol was added into the supernatant so that the final concentration of the ethanol was 50%, and casein phosphopeptide (CPP) was obtained as the precipitate. The phosphopeptide obtained was that similar to Peptide (8) consisting of the position of 1 to 28.

Based on the amino acid sequence of the phosphopeptide obtained, five synthetic phosphopeptides such as the following Peptides (3) to (7), each of which corresponds to the partial sequence of said phosphopeptide, were synthesized. These phosphopeptides were synthesized by the solid phase synthetic method with Fmoc amino acids. In detail, these Peptides were synthesized by repeating the step that α-amino acid of which side chains were protected was bound onto the resin, and then other one was bound to it sequentially. In this case, Fmoc group, 9-Fluorrenylmethoxycarbonyl group, which could be removed by using a strong base, was used as the protection group for α-amino acid. After removing the protection group from the amino acid bound on the resin, the other amino acid was bound on it with the binding agent. Thus obtained peptide was released from the resin by using the strong acid, at the same time removing the protection group for the side chain. Then, the crude peptide obtained was purified by using HPLC to obtain the synthetic peptide of the interest.

Peptide (7) was obtained from Biologica Inc. by requesting to synthesize, and Peptides (3) to (6) were obtained from Becks Co. by requesting to synthesize, respectively. Their purities were 80 % respectively, and the amounts of the peptides were 10mg, respectively.

In addition to said synthetic Peptides (3) to (7), the following three peptides were provided: a peptide consisting of only a phosphoserine (Peptide (2)); control in which neither an amino acid nor a peptide was added (Peptide (1)); and casein phosphopeptide consisting of the position of 1 to 28 of β-casein (Peptide (8)).

Peptide (1):   control

Peptide (2):   SerP alone

Peptide (3):          SerP^15-Leu-SerP^17-NH2

Peptide (4):    Glu14-SerP-Leu-SerP17-                    -Glu21

Peptide (5):                    SerP^17-SerP-SerP-Glu-Glu^21

Peptide (6):    Glu14-SerP-Leu-SerP -SerP18-NH2

Peptide (7):    Glu14-SerP-Leu-SerP -SerP-SerP-Glu-Glu21

Peptide (8):   Arg1-Glu-Leu-Glu-Glu-Leu-Asn-Val-Pro-
Gly-Glu-Ile-Val-Glu14-SerP-Leu-SerP-SerP-SerP-Glu-Glu21-
Ser-Ile-Thr-Arg-Ile-Asn-Lys28

### Evaluation test 1:

Peptides (1) to (8) obtained as above described were subjected to the following evaluation assay to evaluate their immuno enhancing activities.

Murine spleen cells used in the present test were aseptically excised from 6-week-old C3H/HeN strain mail mice. The cells were cultured in Celgrosser-P medium containing 100 U/ml of penicillin and 100 µg/ml of streptomycin.

Each peptide described above was added as a sample to 5 x 10⁶ spleen cells in a micro plate so that the concentration of the peptide was 2nM. Then, 0.5 µg of concanavalin A (ConA), 5 µg of phytohemagglutinin (PHA) or 50 µg of lypopolysaccharide (LPS) were added to the each micro plate, and the cells in each plate were cultured under the condition of 5 % CO₂ at 37°C.

### Test 1A: Cell proliferation assay

The proliferation of the cell was evaluated based on the cell numbers in the cell culture after 48 hours.

After completion of the cell culture, the proliferation of the immune cell was assayed by using dye-MTT method (Mosman, T., J. Immunol. Method, 65 (1983), pp55-63). Formazan generated was determined by using the microplate reader (Bio-Rad Inc., model 450) at absorbance 570 nm.

Significant difference between the control sample and test samples were calculated by using Student's t-test.

The results were shown in Figure 1.

### Test 1B: Immunoglobulin production

The production of immunoglobulin was determined in the cells cultured after 70 to 120 hours.

Amount of immunoglobulin A was measured by using sandwich ELISA method. (Williams, D. J. L. et al., Vet. Immunol. Immunopath., 24 (1990), pp267-283) . Briefly, the cell culture solution was added into wells in a plate coated with swine anti-mouse immunoglobulin A. After the wells were washed, sheep anti-mouse immunoglobulin A antibodies labeled with horseradish peroxidase were added to there to react with said immunoglobulin A. By this means, the amount of immunoglobulin A was determined.

The results were shown in Figure 2.

### Example 2

### Preparation of peptides:

The following synthetic peptides were obtained in the same manner as used in the preparation of Example 1. Peptide (11) was one according to the present invention.

Peptide (9):    Glu14-Ser-Leu-Ser-Ser-Ser-Glu-Glu21

(SEQ. ID No: 13)

Peptide (10):   SerP-SerP

Peptide (11):    SerP17-SerP-SerP19

Peptides (9) to (11) were obtained from Becks Inc. by requesting to synthesize. Their purities were respectively 80%, and the amount of the peptides was 10 mg, respectively.

In addition to said synthetic Peptides (9) to (11), the following two peptides were provided: control in which neither the amino acid nor the peptide was added (Peptide (1)); and casein phosphopeptide consisting of the position of 1 to 28 of β-casein (Peptide (8)).

### Evaluation test 2:

Peptides (9) to (11) obtained as above mentioned, and Peptides (1) and (8) were subjected to the following evaluation assay to evaluate their immuno enhancing activities.

Murine spleen cells used in the present test were provided to be cultured, in the same manner as used in the process of Example 1.

### Test 2A: Cell proliferation activities

The accelerated proliferation of the spleen cells in mouse was evaluated in the same manner as used in the process of Example 1.

The number of the proliferated cells was determined by using MTT values in the same manner as used in the process of Example 1.

The results were shown in Figure 3.

### Test 2B: Immunoglobulin Production

Immunoglobulin production was determined in the cells cultured after 72 to 120 hours.

Amount of immunoglobulins was measured by using sandwich ELISA method. (Williams, D. J. L. et al., Vet. Immunol. Immunopath. , 74(1990), pp323-329) . Briefly, the cell culture solution was added into the wells in the plate coated with goat anti-mouse immunoglobulin (IgG + IgM + IgA or IgA alone). After the wells were washed, sheep goat anti-mouse immunoglobulin antibodies (IgG + IgM + IgA) labeled with horseradish peroxidase or sheep anti-mouse immunoglobulin A similarly labeled with the same were added to there to react with said immunoglobulin A. By this means, the amount of immunoglobulin A was determined.

The results were shown in Figures 4 and 5.

## Claims

1. An immuno-enhancing agent comprising a peptide consisting of the following amino acid sequence (I): Q1-SerP-X-SerP-Q2 (I) wherein,
SerP represents a phosphoserine residue,
X represents 1 to 3 of any amino acid residues, and
either of Q1 or Q2 dose not exist or nei ther of them exists, or Q1 and Q2 are independently at least one of any amino acid residue.

2. The immuno-enhancing agent according to claim 1, wherein X represents any one amino acid.

3. The immuno-enhancing agent according to claim 2, wherein X represents SerP or Leu.

4. The immuno-enhancing agent according to any one of claims 1 to 3, wherein the number of amino acid residues represented by Q1 is 0 to 128, and the number of amino acid residues represented by Q2 is 0 to 192.

5. The immuno-enhancing agent according to any one of claims 1 to 3, wherein the number of amino acid residues represented by Q1 is 0 to 14, and the number of amino acid residues represented by Q2 is 0 to 8.

6. The immuno-enhancing agent according to any one of claims 1 to 5, wherein neither amino acid residues represented by Q1 nor Q2 exists.

7. The immuno-enhancing agent according to claim 5, wherein said amino acid sequence (I) is selected from the group consisting of:

Glu-SerP-X-SerP-SerP-SerP-Glu-Glu (SEQ. ID No: 1);

Glu-SerP-X-SerP-SerP (SEQ. ID No: 2);

SerP-X-SerP-Glu-Glu (SEQ. ID No: 3);
SerP-X-SerP; and

Glu-SerP-X-SerP-Glu (SEQ. ID No: 4).

8. The immuno-enhancing agent according to claim 7, wherein said amino acid sequence (I) is selected from the group consisting of:

Glu-SerP-Leu-SerP-SerP-SerP-Glu-Glu   (SEQ. ID No: 5);

Glu-SerP-Leu-SerP-SerP (SEQ. ID No: 6);

SerP-SerP-SerP-Glu-Glu (SEQ. ID No: 7);
SerP-Leu-SerP;
SerP-SerP-SerP; and

Glu-SerP-Leu-SerP-Glu (SEQ. ID No: 8).

9. The immuno-enhancing agent according to claim 5, wherein said amino acid sequence (I) is

Arg-Glu-Leu-Glu-Glu-Leu-Asn-Val-Pro-Gly-Glu-Ile-Val-Glu-
SerP-X-SerP-SerP-SerP-Glu-Glu-Ser-Ile-Thr-Arg (SEQ. ID
No: 9).

10. The immuno-enhancing agent according to claim 1, wherein said amino acid sequence (I) is 
Arg-Glu-Leu-Glu-Glu-Leu-Asn-Val-Pro-Gly-Glu-Ile-Val-Glu-
SerP-Leu-SerP-SerP-SerP-Glu-Glu-Ser-Ile-Thr-Y-Ile-Asn-
Lys          (SEQ. ID No: 11),
 wherein Y represents any one amino acid residue.

11. The immuno-enhancing agent comprising a peptide which comprises a repetitive sequence in which said amino acid sequence (I) according to any one of claims 1 to 10 is repeated at least twice.

12. The immuno-enhancing agent according to any one of claims 1 to 11 used as pharmaceuticals.

13. The immuno-enhancing agent according to claim 12 further comprising pharmaceutically acceptable additives for pharmaceuticals.

14. The immuno-enhancing agent according to any one of claims 1 to 11 for use as foods.

15. The immuno-enhancing agent according to any one of claims 1 to 11 for use as feeds.

16. A peptide comprising the follwing amino acid sequence (Ia): Q1-SerP-X-SerP-Q2          (Ia) wherein,
SerP represents the phosphoserine residue,
X represents 1 to 3 of any amino acid residues,
Q1 represents 0 to 14 of any amino acid residues,
and
Q2 represents 0 to 8 of any amino acid residues, excluding the case where the number of amino acid in Q2 is 8 and the number of amino acid in Q1 is 14.

17. The peptide according to claim 16, wherein X represents one of any amino acid.

18. The peptide according to claim 17, wherein X represents SerP or Leu.

19. The peptide according to any one of claims 16 to 18, wherein neither amino acid residues represented by Q1 nor Q2 exists.

20. The peptide according to any one of claims 16 to 18, wherein said amino acid sequence (Ia) is selected from the group consisting of:

Glu-SerP-X-SerP-SerP-SerP-Glu-Glu   (SEQ. ID No: 1);

Glu-SerP-X-SerP-SerP (SEQ. ID No: 2);

SerP-X-SerP-Glu-Glu                    (SEQ. ID No: 3);
SerP-X-SerP; and

Glu-SerP-X-SerP-Glu (SEQ. ID No: 4).

21. The peptide according to claim 20, wherein said amino acid sequence (Ia) is selected from the group consisting of:

Glu-SerP-Leu-SerP-SerP-SerP-Glu-Glu   (SEQ. ID No: 5);

Glu-SerP-Leu-SerP-SerP (SEQ. ID No: 6);

SerP-SerP-SerP-Glu-Glu (SEQ. ID No: 7);
SerP-Leu-SerP;
SerP-SerP-SerP; and

Glu-SerP-Leu-SerP-Glu (SEQ. ID No: 8).

22. The peptide comprising a repetitive sequence in which said amino acid sequence (Ia) according to any one of claims 16 to 21 is repeated at least twice.

23. The peptide according to any one of claims 16 to 22, having an immuno enhancing activity.

24. A method for producing the peptide according to any one of claims 16 to 23, comprising the steps of:
providing casein,
treating said casein with trypsin, then precipitating by adding calcium salt and ethanol to obtain phosphopeptide,
treating the phosphopeptide with an enzyme selected from the group consisting of aminopeptidase, carboxypeptidase, and a combination thereof to obtain said peptide.

25. A method for enhancing the immunity of a patient, comprising the step of administrating said peptide according to any one of claims 16 to 23 to the patient with decreased immunity or weakened immunity.

26. Use of said peptide according to any one of claims 16 to 23 for producing an agent for immuno-enhancing.
